# EUROPEAN PATENT APPLICATION

(11) **EP 1 611 900 A1**
(43) Date of publication of application: **04.01.2006**
(21) Application number: 04717780.3
(22) Date of filing: 05.03.2004
(51) Int. Cl.: A61K 38/17, A61K 31/7088, A61K 35/76, A61K 48/00, A61P 19/02, A61P 19/08, A61P 19/10, A61P 29/00, A61P 43/00

(54) **METHOD OF TREATING INFLAMMTORY DISEASE ASSOCIATED WITH BONE DESTRUCTION**

(30) Priority: 19.03.2003 JP 2003075964
(71) Applicant: Dnavec Research Inc., Tsukuba-shi, Ibaraki 305-0856 (JP)
(72) Inventor: SUEISHI, Katsuo, Fukuoka-shi, Fukuoka 8150073 (JP); YONEMITSU, Yoshikazu, Fukuoka-shi, Fukuoka 8130043 (JP); YAMASHITA, Akihisa, Kasuya-gun, Fukuoka 8112311 (JP); YOSHIMURA, Akihiko, Kurume-shi, Fukuoka 8300001 (JP); HASEGAWA, Mamoru, Tsukuba-shi, Ibaraki 3050856 (JP)
(74) Representative: Vossius & Partner
(86) International application number: PCT/JP2004/002887
(87) International publication number: WO 2004/082704

(57) **Abstract**

The present invention relates to methods for treating inflammatory diseases accompanied by bone destruction, comprising the step of administering a viral vector comprising a gene which inhibits signal transduction mediated by fibroblast growth factor-2 (FGF2)-FGF receptor 1-Ras-Raf-MAP kinase to a diseased region. Furthermore, the present invention relates to therapeutic compositions for inflammatory diseases accompanied by bone destruction, which comprise these vectors. By inhibiting FGF2 signal transduction through local administration of viral vectors, both inflammation and bone destruction in inflammatory bone destruction were simultaneously suppressed. The present invention provides disease-specific and effective therapeutic methods, and therapeutic compositions for inflammatory diseases such as osteoarthritis, for which therapy has so far been difficult.

SEQUENCE LISTING

<110>  DNAVEC RESEARCH INC.

<120>  Method of Treating Inflammatory Disease
       Associated With Bone Destruction

<130>  D3-A0206P

<150> JP   2003-075964
<151> 2003-03-19

<160>  6

<170>  PatentIn version 3.1

<210>  1
<211>  900
<212>  DNA
<213>  Homo sapiens

<220>
<221>  CDS
<222>  (1)..(900)
<223>

<400>  1
atg tgg agc tgg aag tgc ctc ctc ttc tgg gct gtg ctg gtc aca gcc        48
Met Trp Ser Trp Lys Cys Leu Leu Phe Trp Ala Val Leu Val Thr Ala
1               5                   10                  15

aca ctc tgc acc gct agg ccg tcc ccg acc ttg cct gaa caa gat gct        96
Thr Leu Cys Thr Ala Arg Pro Ser Pro Thr Leu Pro Glu Gln Asp Ala
            20                  25                  30

ctc ccc tcc tcg gag gat gat gat gat gat gat gac tcc tct tca gag       144
Leu Pro Ser Ser Glu Asp Asp Asp Asp Asp Asp Asp Ser Ser Ser Glu
        35                  40                  45

gag aaa gaa aca gat aac acc aaa cca aac ccc gta gct cca tat tgg       192
Glu Lys Glu Thr Asp Asn Thr Lys Pro Asn Pro Val Ala Pro Tyr Trp
    50                  55                  60

aca tcc cca gaa aag atg gaa aag aaa ttg cat gca gtg ccg gct gcc       240
Thr Ser Pro Glu Lys Met Glu Lys Lys Leu His Ala Val Pro Ala Ala
65                  70                  75                  80

aag aca gtg aag ttc aaa tgc cct tcc agt ggg acc cca aac ccc aca       288
Lys Thr Val Lys Phe Lys Cys Pro Ser Ser Gly Thr Pro Asn Pro Thr
                85                  90                  95

ctg cgc tgg ttg aaa aat ggc aaa gaa ttc aaa cct gac cac aga att       336
Leu Arg Trp Leu Lys Asn Gly Lys Glu Phe Lys Pro Asp His Arg Ile
            100                 105                 110

gga ggc tac aag gtc cgt tat gcc acc tgg agc atc ata atg gac tct       384
Gly Gly Tyr Lys Val Arg Tyr Ala Thr Trp Ser Ile Ile Met Asp Ser
        115                 120                 125

gtg gtg ccc tct gac aag ggc aac tac acc tgc att gtg gag aat gag       432
Val Val Pro Ser Asp Lys Gly Asn Tyr Thr Cys Ile Val Glu Asn Glu
    130                 135                 140

tac ggc agc atc aac cac aca tac cag ctg gat gtc gtg gag cgg tcc       480
Tyr Gly Ser Ile Asn His Thr Tyr Gln Leu Asp Val Val Glu Arg Ser
145                 150                 155                 160

cct cac cgg ccc atc ctg caa gca ggg ttg ccc gcc aac aaa aca gtg       528

Arg Pro Ile Leu Gln Ala Gly Leu Pro Ala Asn Lys Thr Val
165 170 175

ggt agc aac gtg gag ttc atg tgt aag gtg tac agt gac ccg 576
Gly Ser Asn Val Glu Phe Met Cys Lys Val Tyr Ser Asp Pro
180 185 190

cac atc cag tgg cta aag cac atc gag gtg aat ggg agc aag 624
His Ile Gln Trp Leu Lys His Ile Glu Val Asn Gly Ser Lys
195 200 205

cca gac aac ctg cct tat gtc cag atc ttg aag gta atc atg 672
Pro Asp Asn Leu Pro Tyr Val Gln Ile Leu Lys Val Ile Met
215 220

gtc ttc gtg ggc cag tct act ggg aag gag acc act gtc tcg 720
Val Phe Val Gly Gln Ser Thr Gly Lys Glu Thr Thr Val Ser
230 235 240

caa gtt cct gtg ggc agg ctc agt tgc ccc cga atg gga tca 768
Gln Val Pro Val Gly Arg Leu Ser Cys Pro Arg Met Gly Ser
245 250 255

acg ctt cag gca cac aca ctc cat ctc agt agg gat cta gcc 816
Thr Leu Gln Ala His Thr Leu His Leu Ser Arg Asp Leu Ala
260 265 270

ccc agg act agt aac aga ggt cac aaa gtg gag gtg agc tgg 864
Pro Arg Thr Ser Asn Arg Gly His Lys Val Glu Val Ser Trp
275 280 285

agg gct gca ggg atg ggt ggt gct ggt ctg 900
Arg Ala Ala Gly Met Gly Gly Ala Gly Leu
295 300

00
RT
omo sapiens

Ser Trp Lys Cys Leu Leu Phe Trp Ala Val Leu Val Thr Ala
5 10 15

Cys Thr Ala Arg Pro Ser Pro Thr Leu Pro Glu Gln Asp Ala
20 25 30

Ser Ser Glu Asp Asp Asp Asp Asp Asp Asp Ser Ser Ser Glu
35 40 45

Glu Thr Asp Asn Thr Lys Pro Asn Pro Val Ala Pro Tyr Trp
55 60

Pro Glu Lys Met Glu Lys Lys Leu His Ala Val Pro Ala Ala
70 75 80

Val Lys Phe Lys Cys Pro Ser Ser Gly Thr Pro Asn Pro Thr
85 90 95

Trp Leu Lys Asn Gly Lys Glu Phe Lys Pro Asp His Arg Ile
100 105 110

Tyr Lys Val Arg Tyr Ala Thr Trp Ser Ile Ile Met Asp Ser
115 120 125

Pro Ser Asp Lys Gly Asn Tyr Thr Cys Ile Val Glu Asn Glu
135 140
Seite 2

Tyr Gly Ser Ile Asn His Thr Tyr Gln Leu Asp Val Val Glu Arg Ser
145                 150                 155                 160

Pro His Arg Pro Ile Leu Gln Ala Gly Leu Pro Ala Asn Lys Thr Val
                165                 170                 175

Ala Leu Gly Ser Asn Val Glu Phe Met Cys Lys Val Tyr Ser Asp Pro
            180                 185                 190

Gln Pro His Ile Gln Trp Leu Lys His Ile Glu Val Asn Gly Ser Lys
        195                 200                 205

Ile Gly Pro Asp Asn Leu Pro Tyr Val Gln Ile Leu Lys Val Ile Met
    210                 215                 220

Ala Pro Val Phe Val Gly Gln Ser Thr Gly Lys Glu Thr Thr Val Ser
225                 230                 235                 240

Gly Ala Gln Val Pro Val Gly Arg Leu Ser Cys Pro Arg Met Gly Ser
                245                 250                 255

Phe Leu Thr Leu Gln Ala His Thr Leu His Leu Ser Arg Asp Leu Ala
            260                 265                 270

Thr Ser Pro Arg Thr Ser Asn Arg Gly His Lys Val Glu Val Ser Trp
        275                 280                 285

Glu Gln Arg Ala Ala Gly Met Gly Gly Ala Gly Leu
    290                 295                 300

<210>  3
<211>  945
<212>  DNA
<213>  Homo sapiens

<220>
<221>  CDS
<222>  (1)..(945)
<223>

<400>  3
atg gag gcc aga gct cag agt ggc aac ggg tcg cag ccc ttg ctg cag      48
Met Glu Ala Arg Ala Gln Ser Gly Asn Gly Ser Gln Pro Leu Leu Gln
1               5                   10                  15

acg ccc cgt gac ggt ggc aga cag cgt ggg gag ccc gac ccc aga gac      96
Thr Pro Arg Asp Gly Gly Arg Gln Arg Gly Glu Pro Asp Pro Arg Asp
            20                  25                  30

gcc ctc acc cag cag gta cat gtc ttg tct ctg gat cag atc aga gcc      144
Ala Leu Thr Gln Gln Val His Val Leu Ser Leu Asp Gln Ile Arg Ala
        35                  40                  45

atc cga aac acc aat gag tac aca gag ggg cct act gtc gtc cca aga      192
Ile Arg Asn Thr Asn Glu Tyr Thr Glu Gly Pro Thr Val Val Pro Arg
    50                  55                  60

cct ggg ctc aag cct gct cct cgc ccc tcc act cag cac aaa cac gag      240
Pro Gly Leu Lys Pro Ala Pro Arg Pro Ser Thr Gln His Lys His Glu
65                  70                  75                  80

aga ctc cac ggt ctg cct gag cac cgc cag cct cct agg ctc cag cac      288
Arg Leu His Gly Leu Pro Glu His Arg Gln Pro Pro Arg Leu Gln His
                85                  90                  95

tcg cag gtc cat tct tct gca cga gcc cct ctg tcc aga tcc ata agc      336

Ser Gln Val His Ser Ser Ala Arg Ala Pro Leu Ser Arg Ser Ile Ser
            100                 105                 110

acg gtc agc tca ggg tcg cgg agc agt acg agg aca agt acc agc agc      384
Thr Val Ser Ser Gly Ser Arg Ser Ser Thr Arg Thr Ser Thr Ser Ser
        115                 120                 125

agc tcc tct gaa cag aga ctg cta gga tca tcc ttc tcc tcc ggg cct      432
Ser Ser Ser Glu Gln Arg Leu Leu Gly Ser Ser Phe Ser Ser Gly Pro
    130                 135                 140

gtt gct gat ggc ata atc cgg gtg caa ccc aaa tct gag ctc aag cca      480
Val Ala Asp Gly Ile Ile Arg Val Gln Pro Lys Ser Glu Leu Lys Pro
145                 150                 155                 160

ggt gag ctt aag cca ctg agc aag gaa gat ttg ggc ctg cac gcc tac      528
Gly Glu Leu Lys Pro Leu Ser Lys Glu Asp Leu Gly Leu His Ala Tyr
                165                 170                 175

agg tgt gag gac tgt ggc aag tgc aaa tgt aag gag tgc acc tac cca      576
Arg Cys Glu Asp Cys Gly Lys Cys Lys Cys Lys Glu Cys Thr Tyr Pro
            180                 185                 190

agg cct ctg cca tca gac tgg atc tgc gac aag cag tgc ctt tgc tcg      624
Arg Pro Leu Pro Ser Asp Trp Ile Cys Asp Lys Gln Cys Leu Cys Ser
        195                 200                 205

gcc cag aac gtg att gac tat ggg act tgt gta tgc tgt gtg aaa ggt      672
Ala Gln Asn Val Ile Asp Tyr Gly Thr Cys Val Cys Cys Val Lys Gly
    210                 215                 220

ctc ttc tat cac tgt tct aat gat gat gag gac aac tgt gct gac aac      720
Leu Phe Tyr His Cys Ser Asn Asp Asp Glu Asp Asn Cys Ala Asp Asn
225                 230                 235                 240

cca tgt tct tgc agc cag tct cac tgt tgt aca cga tgg tca gcc atg      768
Pro Cys Ser Cys Ser Gln Ser His Cys Cys Thr Arg Trp Ser Ala Met
                245                 250                 255

ggt gtc atg tcc ctc ttt ttg cct tgt tta tgg tgt tac ctt cca gcc      816
Gly Val Met Ser Leu Phe Leu Pro Cys Leu Trp Cys Tyr Leu Pro Ala
            260                 265                 270

aag ggt tgc ctt aaa ttg tgc cag ggg tgt tat gac cgg gtt aac agg      864
Lys Gly Cys Leu Lys Leu Cys Gln Gly Cys Tyr Asp Arg Val Asn Arg
        275                 280                 285

cct ggt tgc cgc tgt aaa aac tca aac aca gtt tgc tgc aaa gtt ccc      912
Pro Gly Cys Arg Cys Lys Asn Ser Asn Thr Val Cys Cys Lys Val Pro
    290                 295                 300

act gtc ccc cct agg aac ttt gaa aaa cca aca                          945
Thr Val Pro Pro Arg Asn Phe Glu Lys Pro Thr
305                 310                 315


<210>  4
<211>  315
<212>  PRT
<213>  Homo sapiens

<400>  4

Met Glu Ala Arg Ala Gln Ser Gly Asn Gly Ser Gln Pro Leu Leu Gln
1               5                   10                  15

Thr Pro Arg Asp Gly Gly Arg Gln Arg Gly Glu Pro Asp Pro Arg Asp
            20                  25                  30

Ala Leu Thr Gln Gln Val His Val Leu Ser Leu Asp Gln Ile Arg Ala
        35                  40                  45

Ile Arg Asn Thr Asn Glu Tyr Thr Glu Gly Pro Thr Val Val Pro Arg
    50                  55                  60

Pro Gly Leu Lys Pro Ala Pro Arg Pro Ser Thr Gln His Lys His Glu
65                  70                  75                  80

Arg Leu His Gly Leu Pro Glu His Arg Gln Pro Pro Arg Leu Gln His
                85                  90                  95

Ser Gln Val His Ser Ser Ala Arg Ala Pro Leu Ser Arg Ser Ile Ser
            100                 105                 110

Thr Val Ser Ser Gly Ser Arg Ser Ser Thr Arg Thr Ser Thr Ser Ser
        115                 120                 125

Ser Ser Ser Glu Gln Arg Leu Leu Gly Ser Ser Phe Ser Ser Gly Pro
    130                 135                 140

Val Ala Asp Gly Ile Ile Arg Val Gln Pro Lys Ser Glu Leu Lys Pro
145                 150                 155                 160

Gly Glu Leu Lys Pro Leu Ser Lys Glu Asp Leu Gly Leu His Ala Tyr
                165                 170                 175

Arg Cys Glu Asp Cys Gly Lys Cys Lys Cys Lys Glu Cys Thr Tyr Pro
            180                 185                 190

Arg Pro Leu Pro Ser Asp Trp Ile Cys Asp Lys Gln Cys Leu Cys Ser
        195                 200                 205

Ala Gln Asn Val Ile Asp Tyr Gly Thr Cys Val Cys Cys Val Lys Gly
    210                 215                 220

Leu Phe Tyr His Cys Ser Asn Asp Asp Glu Asp Asn Cys Ala Asp Asn
225                 230                 235                 240

Pro Cys Ser Cys Ser Gln Ser His Cys Cys Thr Arg Trp Ser Ala Met
                245                 250                 255

Gly Val Met Ser Leu Phe Leu Pro Cys Leu Trp Cys Tyr Leu Pro Ala
            260                 265                 270

Lys Gly Cys Leu Lys Leu Cys Gln Gly Cys Tyr Asp Arg Val Asn Arg
        275                 280                 285

Pro Gly Cys Arg Cys Lys Asn Ser Asn Thr Val Cys Cys Lys Val Pro
    290                 295                 300

Thr Val Pro Pro Arg Asn Phe Glu Lys Pro Thr
305                 310                 315


<210>  5
<211>  1230
<212>  DNA
<213>  Mus musculus

<220>
<221>  CDS
<222>  (1)..(1230)
<223>

<400>  5
atg acc gaa gaa aca cac ccg gac gat gac agc tat att gtg cgt gtc        48

Met Thr Glu Glu Thr His Pro Asp Asp Asp Ser Tyr Ile Val Arg Val
1               5                   10                  15

aag gct gtg gtt atg acc aga gat gac tcc agc ggg gga tgg ttc cca      96
Lys Ala Val Val Met Thr Arg Asp Asp Ser Ser Gly Gly Trp Phe Pro
            20                  25                  30

cag gaa gga ggc ggg atc agt cgc gtc ggc gtg tgt aag gtc atg cac      144
Gln Glu Gly Gly Gly Ile Ser Arg Val Gly Val Cys Lys Val Met His
        35                  40                  45

cct gaa ggc aac gga cga agc ggc ttt ctc atc cat ggc gag cga cag      192
Pro Glu Gly Asn Gly Arg Ser Gly Phe Leu Ile His Gly Glu Arg Gln
    50                  55                  60

aaa gac aaa ctg gtg gta ttg gaa tgc tat gtc aga aag gac ttg gtc      240
Lys Asp Lys Leu Val Val Leu Glu Cys Tyr Val Arg Lys Asp Leu Val
65                  70                  75                  80

tac acc aaa gcc aat ccg acg ttt cat cat tgg aag gtt gat aac agg      288
Tyr Thr Lys Ala Asn Pro Thr Phe His His Trp Lys Val Asp Asn Arg
                85                  90                  95

aag ttt gga ctt act ttc caa agt cct gca gat gca cga gcc ttt gac      336
Lys Phe Gly Leu Thr Phe Gln Ser Pro Ala Asp Ala Arg Ala Phe Asp
            100                 105                 110

agg ggc gtg aga aaa gcc att gaa gac ctt ata gaa ggt tca acg acc      384
Arg Gly Val Arg Lys Ala Ile Glu Asp Leu Ile Glu Gly Ser Thr Thr
        115                 120                 125

tcc tct tcc act ctc cat aac gaa gct gag ctc gga gac gat gac gtt      432
Ser Ser Ser Thr Leu His Asn Glu Ala Glu Leu Gly Asp Asp Asp Val
    130                 135                 140

ttc acg aca gct acg gac agt tct tct aat tcc tcg cag aag agg gag      480
Phe Thr Thr Ala Thr Asp Ser Ser Ser Asn Ser Ser Gln Lys Arg Glu
145                 150                 155                 160

ccg act acg agg aca atc tcc tcc ccc acg tcc tgt gag cac cgg aag      528
Pro Thr Thr Arg Thr Ile Ser Ser Pro Thr Ser Cys Glu His Arg Lys
                165                 170                 175

att tat acc ctt gac cca tac ccc atg gac cat tac cac cct gac cag      576
Ile Tyr Thr Leu Asp Pro Tyr Pro Met Asp His Tyr His Pro Asp Gln
            180                 185                 190

cgg ttg ccg cgg tcc tac ccc cag gtc acc ttc cca gaa gat gat gaa      624
Arg Leu Pro Arg Ser Tyr Pro Gln Val Thr Phe Pro Glu Asp Asp Glu
        195                 200                 205

gaa att gta cgc atc aac ccc cga gag aag atc tgg atg acc ggt tat      672
Glu Ile Val Arg Ile Asn Pro Arg Glu Lys Ile Trp Met Thr Gly Tyr
    210                 215                 220

gaa gac tac cgg cac gcg ccg gtt cgc ggc aaa tac tta gac acc aca      720
Glu Asp Tyr Arg His Ala Pro Val Arg Gly Lys Tyr Leu Asp Thr Thr
225                 230                 235                 240

gaa gac gcg gac tcc tac gtg cgc ttc gcc aag ggc gaa gtc ccc aaa      768
Glu Asp Ala Asp Ser Tyr Val Arg Phe Ala Lys Gly Glu Val Pro Lys
                245                 250                 255

cac gaa tat acc tat ccc tat gtt gat tct tcg gac ttc ggc ttc ggg      816
His Glu Tyr Thr Tyr Pro Tyr Val Asp Ser Ser Asp Phe Gly Phe Gly
            260                 265                 270

gag gat ccc aaa ggt agt gtg atc aag aca cag ccg ccc agg gcc aag      864

Glu Asp Pro Lys Gly Ser Val Ile Lys Thr Gln Pro Pro Arg Ala Lys
        275                 280                 285

tcc cgt cgg cgg aag gag aac ggc gaa cgg tcg cgg tgt gtg tac tgc        912
Ser Arg Arg Arg Lys Glu Asn Gly Glu Arg Ser Arg Cys Val Tyr Cys
    290                 295                 300

agg gat atg ttt aat cac gaa gag aac cga agg ggc cac tgc caa gac        960
Arg Asp Met Phe Asn His Glu Glu Asn Arg Arg Gly His Cys Gln Asp
305                 310                 315                 320

gcg ccc gac gcc gtg aga act tgc att cgc cgg gtg agc tgt atg tgg        1008
Ala Pro Asp Ala Val Arg Thr Cys Ile Arg Arg Val Ser Cys Met Trp
                325                 330                 335

tgc gcg gac agc atg ctg tac cac tgt atg tcc gac ccc gag gga gac        1056
Cys Ala Asp Ser Met Leu Tyr His Cys Met Ser Asp Pro Glu Gly Asp
            340                 345                 350

tac act gac cct tgt tcg tgt gac aca agc gat gag aag ttt tgc ctc        1104
Tyr Thr Asp Pro Cys Ser Cys Asp Thr Ser Asp Glu Lys Phe Cys Leu
        355                 360                 365

cgg tgg atg gct cta att gcc ttg tct ttc ctg gcc cct tgt atg tgc        1152
Arg Trp Met Ala Leu Ile Ala Leu Ser Phe Leu Ala Pro Cys Met Cys
    370                 375                 380

tgt tac ctg ccc ctc cgg gcc tgc cac cgc tgt gga gtg atg tgc agg        1200
Cys Tyr Leu Pro Leu Arg Ala Cys His Arg Cys Gly Val Met Cys Arg
385                 390                 395                 400

tgc tgt ggt ggg aag cac aaa gcc gcc gcg                                1230
Cys Cys Gly Gly Lys His Lys Ala Ala Ala
                405                 410


<210>  6
<211>  410
<212>  PRT
<213>  Mus musculus

<400>  6

Met Thr Glu Glu Thr His Pro Asp Asp Asp Ser Tyr Ile Val Arg Val
1               5                   10                  15

Lys Ala Val Val Met Thr Arg Asp Asp Ser Ser Gly Gly Trp Phe Pro
            20                  25                  30

Gln Glu Gly Gly Gly Ile Ser Arg Val Gly Val Cys Lys Val Met His
        35                  40                  45

Pro Glu Gly Asn Gly Arg Ser Gly Phe Leu Ile His Gly Glu Arg Gln
    50                  55                  60

Lys Asp Lys Leu Val Val Leu Glu Cys Tyr Val Arg Lys Asp Leu Val
65                  70                  75                  80

Tyr Thr Lys Ala Asn Pro Thr Phe His His Trp Lys Val Asp Asn Arg
                85                  90                  95

Lys Phe Gly Leu Thr Phe Gln Ser Pro Ala Asp Ala Arg Ala Phe Asp
            100                 105                 110

Arg Gly Val Arg Lys Ala Ile Glu Asp Leu Ile Glu Gly Ser Thr Thr
        115                 120                 125

Ser Ser Ser Thr Leu His Asn Glu Ala Glu Leu Gly Asp Asp Asp Val

130                 135                 140

Phe Thr Thr Ala Thr Asp Ser Ser Ser Asn Ser Ser Gln Lys Arg Glu
145                 150                 155                 160

Pro Thr Thr Arg Thr Ile Ser Ser Pro Thr Ser Cys Glu His Arg Lys
                165                 170                 175

Ile Tyr Thr Leu Asp Pro Tyr Pro Met Asp His Tyr His Pro Asp Gln
            180                 185                 190

Arg Leu Pro Arg Ser Tyr Pro Gln Val Thr Phe Pro Glu Asp Asp Glu
        195                 200                 205

Glu Ile Val Arg Ile Asn Pro Arg Glu Lys Ile Trp Met Thr Gly Tyr
    210                 215                 220

Glu Asp Tyr Arg His Ala Pro Val Arg Gly Lys Tyr Leu Asp Thr Thr
225                 230                 235                 240

Glu Asp Ala Asp Ser Tyr Val Arg Phe Ala Lys Gly Glu Val Pro Lys
                245                 250                 255

His Glu Tyr Thr Tyr Pro Tyr Val Asp Ser Ser Asp Phe Gly Phe Gly
            260                 265                 270

Glu Asp Pro Lys Gly Ser Val Ile Lys Thr Gln Pro Pro Arg Ala Lys
        275                 280                 285

Ser Arg Arg Arg Lys Glu Asn Gly Glu Arg Ser Arg Cys Val Tyr Cys
    290                 295                 300

Arg Asp Met Phe Asn His Glu Glu Asn Arg Arg Gly His Cys Gln Asp
305                 310                 315                 320

Ala Pro Asp Ala Val Arg Thr Cys Ile Arg Arg Val Ser Cys Met Trp
                325                 330                 335

Cys Ala Asp Ser Met Leu Tyr His Cys Met Ser Asp Pro Glu Gly Asp
            340                 345                 350

Tyr Thr Asp Pro Cys Ser Cys Asp Thr Ser Asp Glu Lys Phe Cys Leu
        355                 360                 365

Arg Trp Met Ala Leu Ile Ala Leu Ser Phe Leu Ala Pro Cys Met Cys
    370                 375                 380

Cys Tyr Leu Pro Leu Arg Ala Cys His Arg Cys Gly Val Met Cys Arg
385                 390                 395                 400

Cys Cys Gly Gly Lys His Lys Ala Ala Ala
                405                 410

## Description

### Technical Field

The present invention relates to methods for treating inflammatory diseases accompanied by bone destruction, by regulating Fibroblast Growth Factor-2 (FGF2) function and inhibiting its intracellular signal transduction system. The methods of the present invention are especially useful for treating Rheumatoid Arthritis (RA).

### Background Art

To date, global research has resulted in the development of various strategies for RA therapy and disease modifying anti-rheumatoid drugs (DMARDs). The leading technologies for RA treatment (RA therapeutic strategies) are listed below. These technologies are treatments and therapeutic strategies using biological agents against RA.

### (1) Therapeutic methods that regulate Tumor Necrosis Factor (TNF)

These are anti-cytokine therapies for treating RA by regulating TNF-α function, using soluble TNF receptors (Moreland, L.W. *et al*., N. Engl. J. Med. 337: 141-147 (1997); Bathon, J.M. *et al*., N. Engl. J. Med. 343: 1586-1593 (2000)) or anti-TNF-α antibodies (Maini, R.N. *et al*., Ann. Rheum. Dis. 58: I56-I60 (1999); Lipsky P.E. *et al*., N. Engl. J. Med. 343:1594-1602 (2000); Maini, R.N. *et al*., Lancet 354: 1932-1939 (1999).

### (2) Therapeutic methods that regulate Interleukin-1 (IL-1)

These are anti-cytokine therapies for treating RA by regulating IL-1 function, using IL-1 receptor antagonists (Cohen, S. *et al*., Arthritis Rheum. 46: 614-624 (2002)).

### (3) Therapeutic methods that regulate IL-6

These are anti-cytokine therapies for treating RA by regulating IL-6 function, using anti-IL-6 receptor monoclonal antibodies (Nishimoto, N. *et al*., Ann. Rheum. Dis. 59: I21-I27 (2000)).

### (4) Therapeutic methods that regulate VEGF function

These are anti-cytokine therapies for treating RA by inhibiting VEGF function using soluble VEGF receptors (Miotla, J. *et al*., Lab. Invest., 80: 1195-205 (2000)) or anti-VEGF antibodies (Lu, J. *et al*., J. Immunol. 164: 5922-7 (2000); Sone, H. *et al*., Biochem. Biophys. Res. Commun. 281: 562-8 (2001)).

However, there are several problems with these conventional technologies. One of the most common problems, for example, is the difficulty in ultimately preventing osteoarticular destruction. That is, although conventional therapies and drugs have a therapeutic effect in terms of relieving subjective and objective symptoms by suppressing synovitis, their effect in directly suppressing osteoarticular destruction is poor. The only effect observed by conventional therapies and drugs is in delaying the progression of bone destruction, thought to be based on the suppression of inflammation, and it is therefore difficult to ultimately avoid osteoarticular destruction. Moreover, in therapies using chimeric neutralizing antibodies, production of human anti-chimeric antibodies is associated with a reduction in the effects of the neutralizing antibodies, and clinically, such therapies must be used together with immune suppressors such as methotrexate. Furthermore, in anti-cytokine therapies targeting inflammatory cytokines, and in systemic administration of biological agents, it is thought probable that various unexpected side effects will arise in unaffected organs.

### Disclosure of the Invention

The present invention provides methods for treating inflammatory diseases accompanied by bone destruction, by inhibiting FGF2 function or blocking its intracellular signal transduction system. Furthermore, the present invention provides therapeutic compositions for diseases, comprising vectors that encode proteins that inhibit FGF2 function or block its intracellular signal transduction system.

FGF2 is a growth factor with an important role in cell differentiation and development (Klansbrun, M., 1989, Prog. Growth Factor Res. 1:207-235; Mason, I., 1994, Cell 78:547-552; Bikfalvi, A. *et al*., 1997, Endocr. Rev. 18:26-45). FGF2 plays an important role in normal developmental processes and tissue regeneration mediated by FGF receptors (FGFRs), and is also involved in cancer growth and inflammation (Basilico, C. and Moscatelli, D, 1992, Adv. Cancer Res. 59:115-165; Klein, S *et al*., 1997, Experientia Suppl. 79:159-192; Jackson, J. *et al*., 1997, FASEB J. 11:457-465). In addition, FGF2 enhances the proliferation of cells such as fibroblasts, vascular endothelium cells, osteoblasts, epidermal cells, and such, and also has a role as a regulatory factor in angiogenesis, bone and cartilage formation, wound healing, and such (Marie, P. *et al*., 2000, Joint Bone Spine 67:150-156; Boyce, B. *et al*., 1999, Lab. Invest. 79:83-94; Goldfarb, M. 1996, Cytokine Growth Factor Rev. 7:311-325; Chikazu, D. *et al*., 2000, J. Biol. Chem. 275:31444-31450; Yamashita, A. *et al*., 2002, J. Immnol. 168:450-457; Collon-Osdoby, P. *et al*., 2002, J. Bone Mineral Res. 17:1859-1871). FGF2 is known to bind to FGFR1 IIIb, FGFR1 IIIc, FGFR2 IIIc, FGFR3 IIIc, and FGFR4 (Ornitz *et al*., 1996, J. Biol. Chem., 271:15292-15297). When FGF2 binds to an FGFR, downstream signal molecules are phosphorylated by FGFR tyrosine phosphorylation activity, and a signal is transduced to Ras, Raf-1, MAPKK, and MAPK. The present inventors considered applying gene therapy, in which this signal transduction is suppressed by a vector that expresses a gene that inhibits signal transduction, to inflammatory bone diseases.

The present inventors constructed viral vectors encoding a secreted form of an FGF2 receptor that inhibits FGF2 function, and Sprouty2 and Spred, which block the intracellular signal transduction system of FGF2. The present inventors then conducted gene therapy by administering the vectors into diseased joints of RA model rats. The results showed that joint inflammation was significantly suppressed in the model rats administered with any of the vectors mentioned above, and furthermore, bone mass reduction was eased and significant therapeutic effect was observed in joints administered with the vectors. Thus, the present inventors succeeded in significantly improving symptoms by simultaneously suppressing inflammation and bone destruction at diseased sites, due to local administration to the diseased bone area of vectors expressing proteins which inhibit FGF2 function or block its intracellular signal transduction system. There are no effective therapeutic methods for inflammatory diseases accompanied by bone destruction, such as RA; however, these new treatments of the methods of the present invention can simultaneously suppress inflammation and bone destruction in a diseased area using gene therapy. Adverse effects caused by systemic administration can be prevented by locally administering vectors to diseased areas. Further, therapeutic effect can be maintained over long periods by the expression of signal transduction inhibitory factors from the vectors.

Therefore, the present invention relates to methods of treating inflammatory diseases accompanied by bone destruction by inhibiting FGF2 function and by inhibiting its cellular signal transduction system. The present invention also relates to pharmaceutical compositions used in these treatments. Specifically, it relates to the inventions described in each item of the Claims. Moreover, the present invention comprises desired combinations of one or more (or all) inventions listed in each item of the Claims. More specifically, the present invention relates to:
[1] A method for treating an inflammatory disease accompanied by bone destruction, comprising the step of administering a vector encoding a protein or a nucleic acid which inhibits a signal transduction that is mediated by fibroblast growth factor-2 (FGF2)-FGF receptor 1-Ras-Raf-MAP kinase.
[2] The method of [1], wherein the vector is a negative single-stranded RNA viral vector.
[3] The method of [2], wherein the negative single-stranded RNA viral vector is a Sendai virus vector.
[4] The method of [1], wherein the protein that inhibits the signal transduction is selected from a group consisting of a soluble FGF receptor, Sprouty2, and Spred.
[5] The method of [1], wherein the disease is osteoarthritis.
[6] A therapeutic composition for an inflammatory disease accompanied by bone destruction, comprising a vector encoding a protein or a nucleic acid which inhibits a signal transduction that is mediated by fibroblast growth factor-2 (FGF2)-FGF receptor 1-Ras-Raf-MAP kinase, and a pharmaceutically acceptable carrier.
[7] The composition of [6], wherein the vector is a negative single-stranded RNA viral vector.
[8] The composition of [7], wherein the negative single-stranded RNA viral vector is a Sendai virus vector.
[9] The composition of [6], wherein the protein that inhibits the signal transduction is selected from a group consisting of a soluble FGF receptor, Sprouty2, and Spred.
[10] The composition of [6], wherein the disease is osteoarthritis.

The present invention provides methods for treating RA by regulating FGF2 function and inhibiting its intracellular signal transduction system for a unified treatment of inflammation and bone destruction in bone diseases. That is, the present inventors provided treatment strategies in which a sequence of the extracellular and/or intracellular FGF2 signal transduction system, mediated by the FGF2-FGF receptor 1-Ras-Raf-MAP kinase system, is inhibited. Inflammation and bone destruction in a diseased area can be suppressed simultaneously, by administering a vector encoding FGF2 signal transduction system inhibitory factors to the diseased area. FGF2 forms a dimer by binding to the FGF receptor (FGFR), and is activated through the induction of tyrosine autophosphorylation. The activated FGFR tyrosine kinase phosphorylates the tyrosine of enzymes such as Src kinase and phospholipase Cγ (PLCγ), as well as the tyrosine of adaptor proteins such as Shc and FGF receptor substrate 2 (FRS2). Phosphorylated Shc and FRS2 bind to Grb2, and recruit Sos to the cell membrane, and Ras is activated by Sos. Activated-type Ras binds to Raf-1 kinase and activates MAPK kinase, which further activates MAP kinase. A tyrosine phosphatase called SHP2 binds to FRS2. Signal transduction mediated by the FGF2-FGF receptor 1-Ras-Raf-MAP kinase is signal transduction that begins from FGF2 and ends at MAPK, as described above. Inhibition of this signal transduction means the inhibition of, for example, one or more desired points in the signal transduction system, from FGF2 to FGF receptor 1, Ras, Raf, and MAP kinase. As an example, FGF2, FGF receptor 1, Shc, Grb2, Sos, FRS2, SHP2, Ras, Raf, MAPKK, and MAPK activities (such as interactions with other signal molecules or phosphorylation activities) may be inhibited. Moreover, expression of each signal molecule (such as transcription, translation, or stability of mRNA-proteins) may be inhibited.

Examples of proteins and nucleic acids which inhibit signal transduction mediated by the FGF2-FGF receptor-1-Ras-Raf-MAP kinase include anti-sense nucleic acids against molecules involved in this signal transduction, RNAs with RNA interference (RNAi) effects, ribozymes, antibodies, and such. Examples of anti-sense nucleic acids include nucleic acids comprising an anti-sense sequence against 13 or more, preferably 14 or more, further preferably 15 or more, and further preferably 20 or more consecutive sense strand nucleotides of a gene encoding a signal molecule. For example, nucleic acids comprising an anti-sense sequence against exon-intron and intron-exon borders in early transcripts, regions comprising a translation initiation codon, and protein-coding sequences in mature mRNAs are preferred. Moreover, examples of ribozymes include ribozymes which cleave the mRNA of target genes. For example, hammerhead-type ribozymes (Rossi, *et al*. (1991) Pharmac. Ther. 50: 245-254) and hairpin-type ribozymes (Hampel, *et al*. (1990) Nucl. Acids Res. 18: 2999-304) are known to cleave specific nucleotide sequences. These ribozymes can use catalytic activity to cleave specific positions in polynucleotides to which an antisense sequence is hybridized. Furthermore, antibodies that bind to domains important for the activity of signal molecules (e.g. phosphorylation sites or sites of interaction with other signal molecules) are used as antibodies that inhibit signal transduction.

Furthermore, in a more preferred embodiment of the present invention, the protein compositions which inhibit signal transduction mediated by the FGF2-FGF receptor 1-Ras-Raf-MAP kinase are expressed by a vector. There are two major preferable means for inhibition: (1) using soluble FGF receptor genes to inhibit signal transduction, and (2) using genes that inhibit the intracellular signal transduction system (Ras-Raf-MAP kinase system). Specific examples of the invention are as follows:
1. Gene therapy using soluble FGF receptor genes
   The current research of various researchers reports that synovial membrane tissues express FGF receptors 1 through 4 and osteoclasts express only receptor 1, and that signal transduction mediated by the FGF receptor 1-MAPK (p42/p44MAPK) is important in osteoclast differentiation and expression of biological function. In preferred embodiments of the present invention, the function of FGF2 is inhibited extracellulary by gene therapy, in which the human soluble FGF receptor (hsFGFR) gene is directly introduced into synovial membrane tissues by a vector. Soluble FGF is a protein comprising an FGF2 binding domain located on the extracellular domain of the FGF receptor, and is secreted outside of cells when expressed within cells. Nucleic acids encoding secretory proteins can be obtained by deleting the nucleic acids encoding the transmembrane and intracellular domains of the FGF receptor gene. Soluble FGF receptors secreted extracellulary inhibit FGF2 signal transduction by binding with FGF2, and by interfering with the binding of FGF2 to endogenous FGFR-1. A significant effect of suppressing inflammation and bone destruction is observed by administering a vector carrying a gene encoding a soluble FGF receptor into damaged bone area. Soluble FGF receptors can be secretory proteins comprising the FGF2 binding region of the FGF receptor which binds FGF2, and they include natural proteins and proteins artificially produced by gene recombination. Specific examples include secretory proteins comprising extracellular domains such as FGFR1a IIIb (Accession NM_015850, NP_056934, AAB19502, Isacchi, A. *et al*., 1990, Nucleic Acids Res. 18:1906; Johnson, D.E. *et al*., 1991, Mol. Cell. Biol. 11:4627-4634), FGFR1b(IIIb)(Accession NM_023106, NP_075594, AAB19502, Isacchi, A. *et al*., 1990, Nucleic Acids Res. 18:1906; Johnson, D.E. *et al*., 1991, Mol. Cell. Biol. 11:4627-4634), FGFR1a(IIIc)(Accession NM_015850, NP_056934, AAB 19502, Isacchi, A. *et al*., 1990, Nucleic Acids Res. 18:1906), FGFR1b(IIIc)(Accession NM_023106, NP_075594, AAB19502, Isacchi, A. *et al*., 1990, Nucleic Acids Res. 18:1906), FGFR2(IIIc)(Accession NM_022962, NP_075251, Johnson, D.E. *et al*., 1991, Mol. Cell. Biol. 11:4627-4634), FGFR3(IIIc)(Accession P22607, Keegan, K. *et al*., 1991, Proc. Natl. Acad. Sci. U.S.A. 88:1095-1099), and FGFR4(Accession AAB25788, Ron, D. *et al*., 1993, J. Biol. Chem. 268:5388-5394). Especially preferably, they include FGFR1 IIIa and human fibroblast growth factor receptor ((FGFr), secreted form), which exist in nature as splice variants, and which function as natural inhibitors of FGF2. For example, the nucleotide sequence (SEQ ID NO: 1) of the gene for the secreted form of FGFr is described in Accession Number M341888, and the amino acid sequence (SEQ ID NO: 2) is described in protein ID AAA35839 (Johnson, D.E. *et al*., Mol. Cell. Biol. 10 (9), 4728-4736 (1990)).
2. Therapy by inhibition of a cellular signal transduction system

The Sprouty2 and Sprouty related Ras signal suppressing factor, Spred, regulates the physiological function of FGF receptor and epidermal growth factor (EGF) by binding to Ras, interfering with the phosphorylation and activation of Raf, and suppressing MAP kinase activation. A therapeutic effect can be achieved by suppressing FGF2 molecule mediated synovitis and osteoclastic bone absorption, by using gene therapy to introduce Sprouty2 or Spred genes into synovial membrane tissues and/or the surrounding skeletal muscle, thereby locally expressing the genes. The nucleotide sequence (SEQ ID NO: 3) and amino acid sequence (SEQ ID NO: 4) of Sprouty2 [*Homo sapiens*] are indicated in Accession Number NM_005842 and NP_005833. The nucleotide sequence (SEQ ID NO: 5) and amino acid sequence (SEQ ID NO: 6) of Spred2 [*Mus musculus*] are indicated in Accession Number AB063496 and BAB62849. Human Spred2 sequences are indicated in Accession Number NM_181784 and NP_861449 (Hacohen, N., *et al*., Cell 92 (2), 253-263 (1998); Glienke, J., *et al*., Mech. Dev. 96 (1), 91-99 (2000); Lim, J., *et al*., J. Biol. Chem. 275 (42), 32837-32845 (2000); Wong, E.S., *et al*., J. Biol. Chem. 276 (8), 5866-5875 (2001); Yusoff, P., *et al*., J. Biol. Chem. 277 (5), 3195-3201 (2002); Egan, J.E., *et al*., Proc. Natl. Acad. Sci. U.S.A. 99 (9), 6041-6046 (2002); Wakioka, T., *et al*., Nature 412 (6847), 647-651 (2001)). In addition, Sef (similar expression to fgf genes), which functions as an antagonist of the FGF signal transduction system mediated by Ras/Raf/MEK/MAPK, can be used (Furthauer, M., *et al*., Nat. Cell Biol., 2002, 4(2):170-4). Moreover, fgf8, fgf3, and Sprouty4 can be also used.

The nucleotide sequence of Sef (similar expression to fgf genes) (also called IL17RLM, FLJ 35755, or IL-17RD) is illustrated between 523 and 2307 of Accession Number NM_017563, and the amino acid sequence of Sef is illustrated in NP_060033 (Clark, H.F., *et al*., Genome Res. 13 (10), 2265-2270 (2003); Yang, R.B., *et al*., J. Biol. Chem. 278 (35), 33232-33238 (2003); Kovalenko, D., *et al*., J. Biol. Chem. 278 (16), 14087-14091 (2003); Furthauer, M., *et al*., Nat. Cell Biol. 4 (2), 170-174 (2002); Tsang, M., *et al*., Nat. Cell Biol. 4 (2), 165-169 (2002)). The nucleotide sequence of FGF8 (fibroblast growth factor 8) gene is illustrated between 237 and 782 of Accession Number NM_033165. The amino acid sequence of FGF8 (mature protein) is illustrated between 23 and 204 of NP_149355 (Gnanapragasam, V.J., *et al*., Br. J. Cancer 88, 1432-1438 (2003); Gnanapragasam, V.J., *et al*., Oncogene 21, 5069-5080 (2002); Tanaka, S., *et al*., Dig. Dis. Sci. 46, 1016-1012 (2001); Ghosh, A.K., *et al*., Cell Growth Differ. 7, 1425-1434 (1996)). The nucleotide sequence of FGF3 (fibroblast growth factor 3) gene is illustrated between 543 and 1208 of Accession Number NM_005247, and the amino acid sequence of FGF3 (mature protein) is illustrated between 18 and 239 of NP_005238 (Kong, M., *et al*., J. Biol. Chem. 277 (18), 15962-15970 (2002); Galdemard, C., *et al*., J. Biol. Chem. 275 (23), 17364-17373 (2000); Thompson, L.M., *et al*., Genomics 11 (4), 1133-1142 (1991)). The nucleotide sequence of Sprouty4 (also called Spry4) gene is illustrated between 188 and 1153 of Accession Number NM_030964, and the amino acid sequence of Sprouty4 is illustrated in NP_112226 (Sasaki, A., *et al*., Nat. Cell Biol. 5 (5), 427-432 (2003); Leeksma, O.C., *et al*., Eur. J. Biochem. 269 (10), 2546-2556 (2002)).

Native proteins with modified amino acids, for example, variants existing in nature and counterparts of other mammals, can be used as the above-mentioned soluble FGFR, and as inhibitory proteins of FGFR intracellular signal transduction. Specifically, proteins that can be preferably used in the present invention include native soluble FGFR or inhibitory proteins of FGFR intracellular signal transduction, comprising an amino acid sequence with one or more amino acid substitutions, deletions, and/or additions; proteins comprising amino acids with 70% or more, preferably 75% or more, more preferably 80% or more, more preferably 85% or more, more preferably 90% or more, and more preferably 95% or more homologous with native soluble FGFR or inhibitory proteins of FGFR intracellular signal transduction; and proteins encoded by nucleic acids which hybridize under stringent conditions with nucleic acids comprising a part of, or the entire coding region of, a native soluble FGFR, or an inhibitory protein of FGFR signal transduction, which inhibit signal transduction mediated by FGF2-FGF receptor 1-Ras-Raf-MAP kinase. For example, a soluble FGFR may comprise desired mutations, as long as it is a secretory protein with FGF2 binding activity. Moreover, mutants of Sprouty2 or Spred can be used in the present invention, as long as they have the activity of inhibiting Ras phosphorylation.

The number of amino acids that are substituted, deleted, and/or added is usually not more than 15, preferably 11 or less, more preferably 9 or less, more preferably 7 or less, and more preferably 5 or less. Proteins comprising amino acids that are conservatively substituted tend to maintain their activity. Conservative substitutions comprise substitutions between amino acids within each of the groups, such as basic amino acids (e.g. lysine, arginine, histidine), acidic amino acids (e.g. aspartic acid, glutamic acid), uncharged amino acids (e.g. glycine, asparagines, glutamine, serine, threonine, tyrosine, cysteine), nonpolar amino acids (e.g. alanine, valine, leucine, isoleucine, proline, phenylalanine, methionine, tryptophan), β-branched amino acids (e.g. threonine, valine, isoleucine), and aromatic amino acids (e.g. tyrosine, phenylalanine, tryptophan, histidine). Amino acid sequence identities can be determined by using, for example, the BLASTP program (Altschul, S. F. *et al*., 1990, J. Mol. Biol. 215: 403-410). Specifically, blastp program may be used. For example, searches may be conducted using default parameters, switching off all filters including Low complexity, at the BLAST web page of NCBI (National Center for Biotechnology Information) (Altschul, S.F. *et al.* (1993) Nature Genet. 3:266-272; Madden, T.L. *et al.* (1996) Meth. Enzymol. 266:131-141; Altschul, S.F. *et al.* (1997) Nucleic Acids Res. 25:3389-3402; Zhang, J. & Madden, T.L. (1997) Genome Res. 7:649-656). For example, by using the BLAST 2 Sequences program (Tatiana A *et al*. (1999) FEMS Microbiol Lett. 174:247-250), which compares two sequences, an alignment of two sequences can be produced to determine identity between the sequences. Gaps are treated similarly to mismatches. For example, an identity value is calculated against the entire amino acid sequence of natural soluble FGFR or FGFR intracellular signal transduction inhibitory protein. Furthermore, in hybridization, probes are prepared from nucleic acids comprising the coding region of natural soluble FGFR or FGFR intracellular signal transduction inhibitory protein, or from nucleic acids subjected to hybridization, and these probes can then be used to identify hybridization by detecting whether they hybridize to other nucleic acids. Stringent hybridization conditions are, for example, hybridization in solutions comprising 5x SSC, 7% (W/V) SDS, 100 µg/ml denatured salmon sperm DNA, 5x Denhardt's solution (1x Denhardt's solution comprises 0.2% polyvinyl pyrrolidone, 0.2% bovine serum albumin, and 0.2% ficoll), at 48°C, preferably at 50°C, and more preferably at 52°C, followed by washing for two hours while shaking at a temperature equal to that of hybridization, preferably 60°C, more preferably 65°C, and most preferably 68°C.

Desired vector systems, including viral vectors and naked DNAs such as plasmid vectors, can be used as the vectors introduced with nucleic acids or genes encoding proteins which inhibit the above mentioned signal transduction. However, viral vectors are especially preferred. Examples of viral vectors include, adenovirus vectors, adeno-associated virus vectors, herpes simplex virus vectors, retrovirus vectors, lentivirus vectors, Semliki forest virus vectors, Sindbis virus vectors, vaccinia virus vectors, fowlpox virus vectors, and Sendai virus vectors. These viral vectors can be prepared as recombinant virus vectors using genetic engineering.

Herein, "recombinant" viral vectors are defined as viral vectors obtained using recombinant polynucleotides, or by amplifying these viral vectors. Furthermore, recombinant polynucleotides are defined as polynucleotides with sequences different to their natural state at either end. More specifically, for example, polynucleotide chains are artificially substituted, or polynucleotides are newly constructed. Recombinant polynucleotides can be produced using known recombinant techniques, by combining methods such as polynucleotide synthesis and nuclease or ligase treatment.

Recombinant viral vectors can be prepared by methods well known to those skilled in the art. For example, the adenovirus vectors used most often in gene therapy can be produced by the methods of Saito *et al.* and others (Miyake, *et al.,* 1996, Proc. Natl. Acd. Sci. USA, vo1.93, 1320-24; Kanegae, *et al*., 1996, Acta Paediatr. Jpn, vol. 38, 182-188; Kanegae, *et al*., Biomannual Series 4- Idensi dounyu-to hatsugen kaisekihou (Gene Transfer and Expression-Analysis), 1994, 43-58, Yodosha; Kanegae, *et al*., 1994, Saibokogaku (Cell Technology), vo1.13. Number 8. 757-763). Furthermore, for example, retrovirus vectors (Wakimoto, *et al*., 1995, Tanpakushitsu Kakusan Koso (Protein, Nucleic Acids, and Enzyme), vol 40, 2508-2513) and adeno-associated virus vectors (Tamaki *et al*., 1995, Tanpakushitsu Kakusan Koso (Protein, Nucleic Acids, and Enzyme), Vol. 40, 2532-2538) can be also produced by well known methods. Detailed descriptions for producing other viral vectors that can transfer genes into mammals are shown in the Japanese Patent Kohyo Publication No. (JP-A)H6-502069 (unexamined Japanese national phase publication corresponding to a non-Japanese international publication), Japanese Patent Application Kokoku Publication No. (JP-B) H6-95937 (examined, approved Japanese patent application published for opposition), and JP-B H6-71429 for producing a recombinant vaccinia virus. Methods for producing recombinant Papiloma viruses are shown in JP-B H6-34727 and JP-A H6-505626. Methods for producing recombinant adeno-associated viruses are shown in JP-A H5-308975. Methods for constructing recombinant adenoviruses are shown in JP-A H6-508039.

Of the viral vectors, negative single-stranded RNA viruses are especially preferred vectors in the present invention. Negative single-stranded viral vectors refer to vectors (carriers) that transfer genes into host cells, where the viruses or their derivatives possess a negative strand [i.e., a (-) strand] of single-stranded RNA in their genomes. Negative single-stranded RNA viruses include viruses which belong to families such as Paramyxoviridae (including the genera paramyxovirus, Morbillivirus, Rubulavirus, Pneumovirus, and such), Rhabdoviridae (including the genera Vesiculovirus, Lyssavirus, Ephemerovirus, and such), Firoviridae, Orthomyxoviridae (including the Influenza virus A, B, and C, Thogoto-like viruses, and such), Bunyaviridae (including the genera Bunyavirus, Hantavirus, Nairovirus, Phlebovirus, and such), and Arenaviridae. Viral vectors belonging to Paramyxoviridae (herein referred to as paramyxovirus vectors) are especially preferred. The present inventors revealed that administration of paramyxovirus vectors encoding FGF2 signal inhibitory factor significantly decreases both inflammation of bone destructive arthritis and bone mass reduction. Paramyxovirus vectors are RNA viral vectors that do not integrate genes into the chromosome, and have the characteristic of expressing foreign genes at extremely high levels for a certain period of time. The characteristics of these vectors are considered to cause especially preferable effects in treating bone destructive inflammatory diseases in the methods of the present invention.

The paramyxoviruses used in the present invention include, for example, viruses belonging to the *Paramyxoviridae* family, such as Sendai virus, Newcastle disease virus, mumps virus, measles virus, respiratory syncytial virus, rinderpest virus, distemper virus, simian parainfluenza virus (SV5), and type I, II, and III human parainfluenza virus. Preferred viruses of the present invention include those belonging to the subfamily Paramyxovirinae (including the genera of Respirovirus, Rubulavirus, and Mobillivirus), more preferably the genus Respirovirus (also called paramyxovirus) or derivatives thereof. Respiroviruses that can be used in the present invention include, for example, type I human parainfluenza virus (HPIV-1), type III human parainfluenza virus (HPIV-3), type III bovine parainfluenza virus (BPIV-3), Sendai virus (also called type I mouse parainfluenza virus), and type X simian parainfluenza virus (SPIV-10). A most preferable paramyxovirus of the invention is Sendai virus. These viruses may be naturally occurring, wild-type, mutant, laboratory-passaged, or artificially constructed strains, etc. Incomplete viruses such as the DI particle (Willenbrink, W. and Neubert, W.J., J. Virol. 68: 8413-8417 (1994)) and synthesized oligonucleotides may be utilized as materials for generating the viral vectors of the present invention.

Recombinant negative strand RNA viral vectors can be generated by constructing recombinant DNAs in which an appropriate transcription promoter is connected to a DNA encoding the viral genome, transcribing these DNAs in test tubes or cells, reconstructing RNP in the presence of the L, P, and NP proteins of negative strand RNA viruses, and finally forming viral particles comprising the above mentioned RNP. Specifically, they can be normally prepared by (a) transcribing a vector DNA encoding a negative single-stranded RNA derived from a negative stranded RNA virus or its complementary (positive) strand in cells (helper cells) that express NP, P, and L proteins, and (b) culturing the cells and recovering viral particles from the culture supernatant. RNAs transcribed from the vector DNAs form RNP complexes with NP, L, and P proteins, and viral particles wrapped in a coat comprising envelope protein are formed. Viruses can be reconstructed from viral vector DNAs using well known methods (Hasan, M. K. *et al.,* J. Gen. Virol. 78: 2813-2820, 1997, Kato, A. *et al*., 1997, EMBO J. 16: 578-587; Yu, D. *et al*., 1997, Genes Cells 2: 457-466; WO 97/16539; WO 97/16538; Durbin, A. P. *et al*., 1997, Virology 235: 323-332; Whelan, S. P. *et al*., 1995, Proc. Natl. Acad. Sci. USA 92: 8388-8392; Schnell. M. J. *et al*., 1994, EMBO J. 13: 4195-4203; Radecke, F. *et al*., 1995, EMBO J. 14: 5773-5784; Lawson, N. D. *et al*., Proc. Natl. Acad. Sci. USA 92: 4477-4481; Garcin, D. *et al*., 1995, EMBO J. 14: 6087-6094; Kato, A. *et al*., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404). Using these methods, desired paramyxovirus vectors comprising parainfluenza, vesicular stomatitis virus, rabies virus, measles virus, rinderpest virus, Sendai virus, and other negative strand RNA viral vectors may be reconstructed from DNA.

Negative strand RNA viruses express an extremely high amount of protein, and can transfer genes into various types of cells. Moreover, Sendai virus (SeV) in particular does not show serious harmful effects in primates, and is preferably used for human gene therapies in the present invention (Hurwitz, J. L. *et al*., Vaccine 15: 533-540, 1997).

Viral vectors may be derivatives of wild type viruses. Derivatives refer to vectors in which the viral gene or viral protein carried is modified without completely impairing the viral ability to transfer a gene. These modifications can be performed to destroy the viral replication ability or to reduce viral antigenicity. For example, replication deficient viral vectors, in which a gene necessary for viral propagation is deleted, can be used. When F gene, HN gene, and/or M gene and such, are deleted from a paramyxovirus virus vector DNA, infectious viral particles will not form in this state. However, infectious viral particles can be formed by separately transferring and expressing these deleted genes, and/or genes encoding envelope protein from other viruses, in host cells. Methods for producing these deleted-form viral vectors are known, and thus, the vectors can be produced according to or by following these methods (see International Publication No. WO 00/70055 and WO 00/70070). The expression levels of foreign genes from vectors can be regulated by the type of transcription initiation sequence ligated upstream of the gene, for example (International Publication No. WO 01/18223). Furthermore, the viral vectors may be pseudotyped viral vectors comprising an envelope protein derived from another virus. These envelope proteins include VSV-G (see International Publication No. WO 00/70055 and WO 00/70070).

Furthermore, accessory genes possessed by the virus may be deleted. For example, the pathogenicity of SeV against hosts such as mice is significantly reduced by knocking out the V gene, one of the SeV accessory genes, without disturbing gene expression and replication in cultured cells (Kato, A. *et al*., 1997, J. Virol. 71: 7266-7272; Kato, A. *et al*., 1997, EMBO J. 16: 578-587; Curran, J. *et al*., WO 01/04272, EP 1067179). These attenuated vectors are especially useful as viral vectors for low toxicity *in vivo* or *ex vivo* gene transfer.

The collected viral vectors may be purified to be substantially pure. Purification may be performed by known purification and separation methods, and combinations thereof, including filtration, centrifugation, and column purification. "Substantially pure" means that the viral vector accounts for the majority of the components present in a sample. Typically, viral vectors are substantially pure when proteins originating from the viral vectors account for 10% or more, preferably 20% or more, more preferably 50% or more, preferably 70% or more, more preferably 80% or more, and even more preferably 90% or more of the proteins comprised in a sample (excluding those proteins which were added as carriers and stabilizers). Purification methods for paramyxovirus include, for example, methods that use cellulose sulfate or cross-bridged polysaccharide sulfate (Japanese Patent Application Kokoku Publication No. (JP-B) S62-30752, JP-B S62-33879, and JP-B 62-30753) and methods that use adsorption to polysaccharides comprising fucose sulfate and/or its decomposed product (WO 97/32010).

Viral titers can be determined, for example, by measuring CIU (Cell-Infected Units) or hemagglutination activity (HA) (WO 00/70070; Kato, A. *et al*., 1996, Genes Cells 1: 569-579; Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Titers of vectors carrying GFP (green fluorescent protein) marker genes and the like can be quantified by directly counting infected cells, using the marker as an indicator (for example, as GFP-CIU). Titers thus measured can be handled in the same way as CIU (WO 00/70070). Vectors thus obtained are drugs for preventing and treating inflammatory diseases accompanied by bone destruction. In particular, the above-mentioned vectors are useful as drugs for preventing and treating rheumatoid arthritis.

Vectors can be combined with desirable pharmaceutically acceptable carriers or media as necessary, for producing compositions comprising viral vectors. "Pharmaceutically acceptable carriers or media" refer to materials that can be administered with a vector and do not significantly inhibit gene transfer by the vector. Specifically, the vectors may be appropriately combined with sterile water, physiological saline, culture media, phosphate buffered saline (PBS) and such, to formulate drugs. Furthermore, vegetable oils, suspension agents, surfactants, stabilizers, sterilizers and such, can also be included in addition to the materials described above. A composition of the present invention may be in the form of a solution, capsule, suspension, syrup, and such. Compositions comprising the produced viral vectors are useful as reagents or pharmaceutical agents. The compositions are used, for example, as reagents or pharmaceutical agents to suppress osteoclast formation, or bone destruction and/or inflammation, or as pharmaceutical agents to prevent or treat bone destructive diseases.

The symptoms of a patient with an inflammatory disease accompanied by bone destruction may be significantly reduced by administering a vector, produced as described above, into an affected area. Herein, "inflammatory diseases accompanied by bone destruction" refers to diseases accompanied by destruction, reduction, absorption, and/or osteoporosis and inflammation of a bone. Examples of these diseases specifically include rheumatoid arthritis (RA). Inflammation and bone destruction caused by rheumatoid arthritis can be suppressed using the methods of the present invention. Furthermore, treatment for diseases including juvenile rheumatoid arthritis, psoriatic arthritis, and multiple arthritis, which frequently arises in various connective tissue diseases, are also subjects of the present invention.

RA is a persistent inflammation in the joint synovial membrane. RA proliferation and hyperplasia result in bone destruction in joint cartilage and its surroundings, causing chronic disease associated with joint cartilage deformity and bone destruction. The present invention provides methods for treating RA by regulating FGF2 function and inhibiting its intracellular signal transduction system, as a unified treatment for synovitis and bone destruction due to RA. That is, the present inventors provided extracellular and intracellular inhibition of the sequential FGF2 signal transduction system, mediated by the FGF2-FGF receptor 1-Ras-Raf-MAP kinase system, as a treatment strategy for RA.

### 1. Suppression of RA synovitis

The main constituent of RA synovialis is proliferative synovitis. Blood vessels newly formed by the pathophysiological phenomenon called (morbid) angiogenesis are important in proliferative synovitis for the formation and development of RA symptoms and bone destruction in terms of (1) the route to supply nutrients to synovial membrane tissues, (2) the route of access to inflammatory cells, (3) the route of access to osteoclast progenitor cells, and (4) the route of access to disease mediators such as cytokines. Therefore, RA treatment strategies that regulate the function of angiogenesis factors are suitable. Inflammation is thought to be suppressed as the vascular bed in the synovial membrane is decreased. Moreover, the present invention is considered to display a more selective and stronger anti-synovitis effect, since it aims to functionally regulate FGF2, which is a particularly harmful factor among the angiogenesis factors that cause inflammation and bone destruction in RA.

### 2. Suppression of RA bone destruction

As mentioned before, integrated and effective treatment of arthritis and bone destruction using conventional technology has been difficult. It is reported that FGF2, a target molecule in the present invention, not only has biological function as an angiogenic factor, but also as a differentiation factor and function enhancer for osteoclasts, which play a central role in bone destruction. Therefore, by regulating FGF2 function, differentiation and function of osteoclasts, as well as the development of synovitis, can be directly suppressed. Thus, it is possible to suppress both the phenomenon of synovitis and of bone joint destruction, which is the ultimate target for RA treatment.

### 3. Drug delivery systems

When a protein drug known to be effective for RA is systemically administered orally or intravenously, important issues remain, such as: (1) delivery of drugs to the joint synovial membrane area, (2) obtaining effective concentrations in the area, (3) adverse effects in various organs. On the other hand, in the gene therapy methods of the present invention, effective expression levels of genes can be maintained in the joint area for a specific period of time by expressing therapeutic genes in synovial membrane tissues using a vector. By using these gene therapy techniques, it is possible to prevent proteins from becoming highly concentrated, locally or systemically.

### 4. Biological drug development technologies

The present invention comprises methods for administering a vector introduced with genes which suppress FGF2 function or genes which block an intracellular signal transduction system downstream of the FGF2 receptor. That is, therapeutic drugs and treatments that are more disease- and symptom-specific can be produced by selectively inhibiting a single enzyme in the intracellular signal transduction system. Thus, the present invention makes possible desirable biological formulations that selectively inhibit disease-specific molecules.

The doses of the vectors may vary depending on the disease, the patient's body weight, age, sex, symptoms, purpose of administration, form of administrative composition, method of administration, transgene, and so on, but they can be appropriately determined by one skilled in the art. The administration route can be properly determined, however, local injection is preferable in order to specifically reach disease areas with bone destroying inflammation, or administration using a proper drug delivery system is preferable to reach disease areas. When a paramyxovirus vector is used, it is preferable to administer with the vector concentration preferably in the range of approximately 10⁵ CIU/ml to approximately 10¹¹ pfu/ml, more preferably approximately 10⁷ CIU/ml to approximately 10⁹ CIU/ml, and most preferably approximately 1 x 10⁸ CIU/ml to approximately 5 x 10⁸ CIU/ml, in pharmaceutically acceptable carriers. The administered dose may be preferably in the range of approximately 10⁵ pfu to 10¹¹ pfu, more preferably approximately 10⁷ pfu to 10⁹ pfu, and most preferably approximately 10⁸ pfu to 10⁹ pfu per area. When a combination of multiple vectors is administered, each vector is desirably administered at a dose described above. Administration may be performed at single or multiple (2, 3, 4 and 5 to 10) sites. In cases of *ex vivo* administration, cells and such collected from patients are contacted with a vector outside of the body (for example, in a test tube or in a Petri dish). An MOI of between 1 to 500, more preferably 2 to 300, and even more preferably 3 to 200 is preferable for administration. The cells are then injected into patients. The compositions comprising the vectors of the present invention may be administered to subjects including humans and all non-human mammalian animals, including monkeys, mice, rats, rabbits, sheep, cattle, dogs, and such.

### Brief description of the Drawings

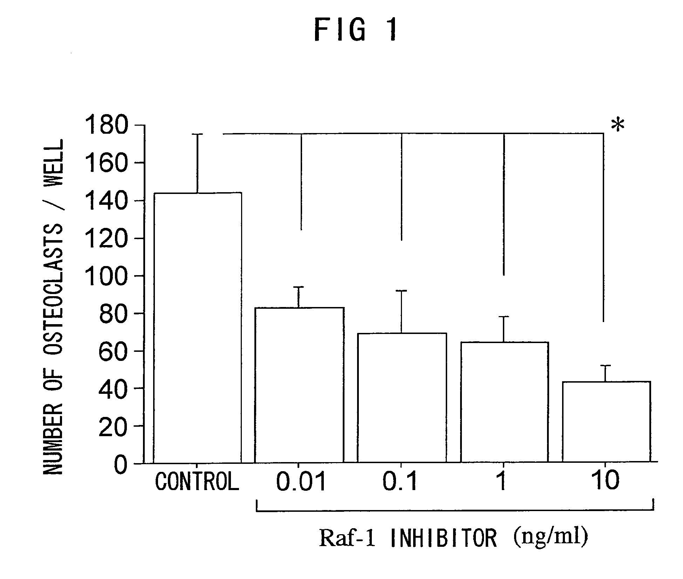

Fig. 1 is a graph showing the suppression of osteoclast formation by administration of Raf inhibitor. The results are expressed as a mean ± standard deviation. Statistical analysis was conducted using one-way ANOVA. (*) Significance levels of p<0.01 were determined to be significantly different (n=8 culture groups each).

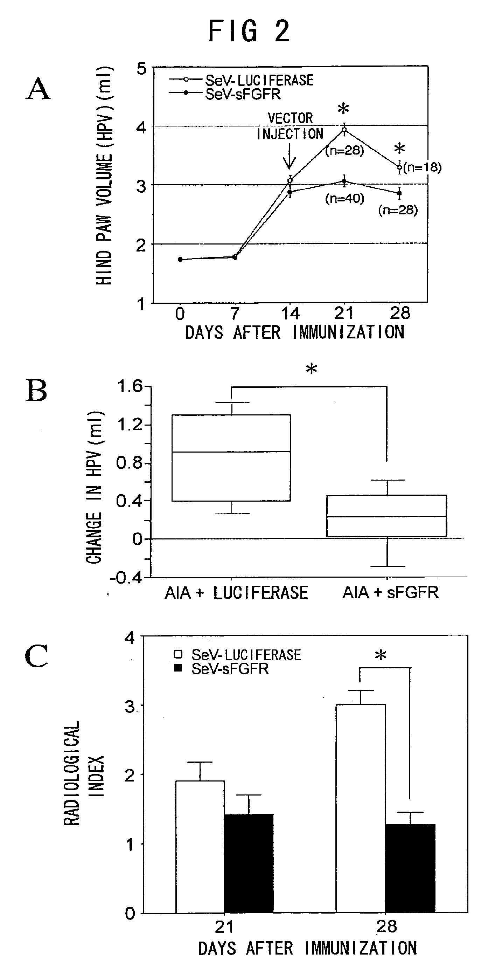

Fig. 2 is a graph showing the efficacy of AIA treatment by sFGFR gene transfer. Vector injection in the graph indicates the time when SeV carrying the sFGFR gene was administered to the foot-pad. The results are shown as a mean ± standard error. Statistical analysis was conducted using Mann-Whitney U-tests. (*) Significance levels of p<0.01 were determined to be significantly different.

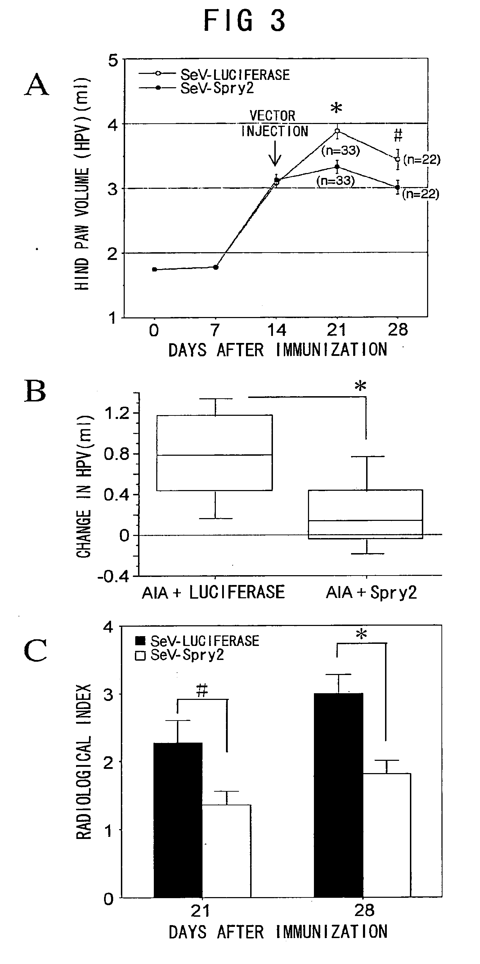

Fig. 3 is a graph showing the efficacy of AIA treatment by spry2 gene transfer. Vector injection in the graph indicates the time when SeV carrying the spry2 gene was administered to the foot-pad. The results are shown as a mean ± standard error. Statistical analysis was conducted using Mann-Whitney U-tests. (#) Significance levels of p<0.05 and (*) significance levels of p<0.01 were determined to be significantly different.

### Best Mode for Carrying out the Invention

The present invention is specifically illustrated below with reference to Examples, but it is not to be construed as being limited thereto. Furthermore, the references cited throughout this description are incorporated herein by reference.

### [Example 1] Effect of Raf inhibitor on cultured osteoclast formation

### 1. Isolation culture for rat bone marrow derived macrophages

Isolation culture for rat bone marrow derived macrophages was performed using macrophage colony stimulating factor (M-CSF). Eight Charles-River grade Lewis rats (six weeks old, KBT Oriental Co., Ltd., Tosu, Saga) were used, and sacrificed by cervical dislocation under anesthesia, and the carotid artery was dissected to remove blood. Bone stem from both the femur and tibia was removed under sterile conditions, and myeloma cells were collected by injecting culture media (α-modified essential medium: α-MEM (GIBCO BRL, Rockville, Maryland, USA) into the medullary cavity of the bone. Erythrocytes were washed with 0.727% NH₄Cl - 0.017% Tris-Cl(pH7.2) - phosphate buffered saline (PBS) solution, and then 5 x 10⁶ cells were dispersed in a 180 ml flask using α-MEM comprising 10% fetal bovine serum (FBS) and recombinant human M-CSF (rhM-CSF, 10 ng/ml, CHEMICON International, Inc. Temecula, CA). After washing with PBS after three days, adhesive cells were detached using PBS comprising 0.05% trypsine/0.02% EDTA, and 10⁶ cells were plated in a 180 ml flask. Cells were similarly collected after three days, then stored in liquid nitrogen. Rat bone marrow derived macrophages isolated by the method described above were defined as M-CSF-dependent bone marrow macrophages (MDBM).

### 2. Effect of Raf inhibitor on osteoclast formation

MDBM were seeded onto a 96 well plate at 5 x 10³ cells/well, using pre-prepared α-MEM, comprising 10% FBS, rhM-CSF (10 ng/ml), recombinant human soluble receptor activator of nucleus factor-kappa B ligand (sRANKL, 50 ng/ml, PeproTech EC, Ltd. London, U.K.), and recombinant human FGF basic (rhFGF-2, 10 ng/ml, Genzyme/Techne, Minneapolis, MN, USA). Raf inhibitor (Raf-1 kinase Inhibitor I, Calbiochem, San Diego, CA, USA) dissolved in dimethylsulfoxide (DMSO) was added (10 µl/well) after attachment of cells. The same amount of DMSO alone was added to the control group. The culture media was exchanged after three days and Raf inhibitor was added again. Seven days after the culture was started, tartrate-resistant acid phosphatase (TRAP) staining was performed using a leukocyte acid phosphatase kit (Sigma Chemical Co., St. Louis, MO, USA). TRAP-positive multinucleated cells comprising three or more nuclei were defined as osteoclasts, and the number of formed osteoclasts was compared and examined. The results showed that administration of Raf inhibitor suppressed osteoclast formation concentration dependently, and suppression was significantly different to the control at any concentration (Fig. 1).

### [Example 2] Effect ofsFGFR gene transfer on AIA: extracellular blocking of FGF-2

### <Method>

### 1. AIA model

For the AIA model, rats were injected subcutaneously at the base of the tail with 1 mg Mycobacterium Butyricum Desiccated (MBD, Difco, Detroit, MI, USA) dissolved in 100 µl mineral oil (NACALAI TESQUE, Kyoto).

### 2. Experimental protocol for extracellular blocking of FGF2

The target gene was expressed in rats at the inflammation site by direct gene transfer methods using recombinant Sendai virus vector (SeV), and the effect on arthritis was examined. Specifically, after arthritis development was confirmed, SeV carrying soluble FGF receptor gene (sFGFR, SEQ ID NO: 1) (SeV-sFGFR) was administered to the foot-pad at 10⁸ pfu/foot in the AIA + sFGFR group (n=40) 14 days after adjuvant administration. SeV carrying the firefly luciferase gene (SeV-luciferase) was administered to the foot-pad at 10⁸ pfu/foot in AIA + luciferase control group (n=28) 14 days after adjuvant administration.

### 3. Measurement of hind paw volume

Hind paw volume (HPV) was measured sequentially using a volume meter (MK-550; MUROMACHI KIKAI CO., LTD., Tokyo) to quantify the extent of swelling in the hind paw caused by arthritis.

### 4. Radiological assessment of bone and joint destruction

To radiologically examine bone and joint destruction, rats were sacrificed and their hind paws amputated at the middle of the lower leg. Then, the extent of bone and joint destruction was evaluated radiologically by soft ray radiography, using a scale of 5 (CMB-2; Softex Corporation, Tokyo) at days 21 and 28. The radiological bone and joint destruction index (Radiological Index: RI) for the present Example is as follows: 0: no change, 1: mild bone atrophy and expansion of shadow in cartilage, 2: narrowing of joint space and moderate bone atrophy, 3: bone and cartilage erosion or moderate joint destruction, 4: loss of joint structure due to severe bone destruction.

### <Result>

Fig. 2A shows a time course of hind paw volume. Hind paw volume was 3.06 ± 0.1 in the AIA + sFGFR group and 3.93 ± 0.11 ml in the AIA + luciferase group, at day 21. Hind paw volume was 2.85 ± 0.1 in the AIA + sFGFR group and 3.29 ± 0.12 ml in the AIA + luciferase group, at day 28. Hind paw swelling was significantly suppressed by blocking of the FGF-2 extracellular signal by sFGFR gene transfer at both time points. Fig. 2B shows the rate in volume change between days 14 and 21. The results showed that the rate was 0.18 ± 0.07 in the AIA + sFGFR group and 0.86 ± 0.09 ml in the AIA + luciferase group. Therefore, the rate in volume change was also significantly suppressed by blocking of FGF-2 extracellular signal seven days after vector administration.

Fig. 2C shows the radiological index for bone and joint destruction. The indices were 1.42 ± 0.29 (n=12) in the AIA + sFGFR group and 1.9 ± 0.28 (n=10) in the AIA + luciferase group, at day 21. The indices were 1.27 ± 0.18 (n=26) in the AIA + sFGFR group and 3.0 ± 0.21 (n=14) in the AIA + luciferase group, at day 28. Therefore, bone and joint destruction was significantly suppressed by blocking the FGF-2 extracellular signal at both time points.

### [Example 3] Effect of Spry2 gene transfer on AIA: Blocking of FGF-2 intracellular signal

### <Method>

### 1. AIA model

Rats were injected subcutaneously at the base of the tail with 1 mg Mycobacterium Butyricum Desiccated (MBD, Difco) dissolved in 100 µl mineral oil (NACALAI TESQUE).

### 2. Experimental protocol for intracellular FGF-2 blocking

The target gene was expressed at the inflammation site in rats by a direct gene transfer method using recombinant Sendai virus vector (SeV), and the effect on arthritis was examined. Specifically, after arthritis development was confirmed, SeV carrying human Sprouty2 gene (Spry2: SEQ ID NO: 3) (SeV-spry2) was administered to the foot-pad at 10⁸ pfu/foot in the AIA + Spry2 group (n=33) at 14 days after adjuvant administration. SeV carrying firefly luciferase gene (SeV-luciferase) was administered to the foot-pad at 10⁸ pfu/foot in the AIA + luciferase control group (n=33) at 14 days after adjuvant administration.

### 3. Measurement of hind paw volume

Hind paw volume (HPV) was sequentially measured using a volume meter (MK-550; MUROMACHI KIKAI) to measure the extent of hind paw swelling caused by arthritis.

### 4. Radiological assessment of bone and joint destruction

To radiologically examine bone and joint destruction, rats were sacrificed and their paws amputated at the middle of the lower leg. Then, the extent of bone and joint destruction was evaluated radiologically by soft ray radiography, using a scale of 5 (CMB-2; Softex Corporation) at days 21 and 28. The radiological bone and joint destruction index (Radiological Index: RI) for the present Example was as follows: 0: no change, 1: mild bone atrophy and expansion of shadow in cartilage, 2: narrowing of joint space and moderate bone atrophy, 3: bone and cartilage erosion or moderate joint destruction, 4: loss of joint structure due to severe bone destruction.

### <Result>

Fig. 3A shows a time course of hind paw volume. Hind paw volume was 3.33 ± 0.1 in the AIA + Spry2 group and 3.85 ± 0.12 ml in the AIA + luciferase group at day 21. Hind paw volume was 3.07 ± 0.12 in the AIA + Spry2 group and 3.38 ± 0.16 ml in the AIA + luciferase group at day 28. Hind paw swelling was significantly suppressed by blocking FGF-2 intracellular signaling mediated by FGFR 1/Ras/Raf/MAPK by Spry2 gene transfer at both time points.

Fig. 3B shows the rate of volume change between days 14 and 21. The results showed that the rate was 0.21 ± 0.07 in the AIA + Spry2 group and 0.77 ± 0.08 ml in the AIA + luciferase group. Therefore, the rate of volume change due to blocking FGF-2 intracellular signaling was also significantly suppressed seven days after administration of vector.

Fig. 3C shows the radiological index for bone and joint destruction. The indices werel.36 ± 0.20 (n=11) in the AIA + Spry2 group and 2.27 ± 0.33 (n=11) in the AIA + luciferase group, at day 21. The indices were 1.82 ± 0.19 (n=22) in the AIA + Spry2 group and 3.0 ± 0.28 (n=22) in the AIA + luciferase group, at day 28. Therefore, the bone and joint destruction due to blocking of FGF-2 intracellular signaling was significantly suppressed at both time points.

### Industrial Applicability

Based on the concept of "functional inhibition of FGF2 or its signal transduction system", the present invention makes possible biological drug formulations that are more disease-specific, effective, and safe. Moreover, the present invention, which targets angiogenesis factor FGF2 molecules in its RA treatment strategies, establishes effective therapeutic methods to not only suppress arthritis, but also to suppress disease progression, and to prevent bone destruction.

## Claims

1. A method for treating an inflammatory disease accompanied by bone destruction, comprising the step of administering a vector encoding a protein or a nucleic acid which inhibits a signal transduction that is mediated by fibroblast growth factor-2 (FGF2)-FGF receptor 1-Ras-Raf-MAP kinase.

2. The method of Claim 1, wherein the vector is a negative single-stranded RNA viral vector.

3. The method of Claim 2, wherein the negative single-stranded RNA viral vector is a Sendai virus vector.

4. The method of Claim 1, wherein the protein that inhibits the signal transduction is selected from a group consisting of a soluble FGF receptor, Sprouty2, and Spred.

5. The method of Claim 1, wherein the disease is osteoarthritis.

6. A therapeutic composition for an inflammatory disease accompanied by bone destruction, comprising a vector encoding a protein or a nucleic acid which inhibits a signal transduction that is mediated by fibroblast growth factor-2 (FGF2)-FGF receptor 1-Ras-Raf-MAP kinase, and a pharmaceutically acceptable carrier.

7. The composition of Claim 6, wherein the vector is a negative single-stranded RNA viral vector.

8. The composition of Claim 7, wherein the negative single-stranded RNA viral vector is a Sendai virus vector.

9. The composition of Claim 6, wherein the protein that inhibits the signal transduction is selected from a group consisting of a soluble FGF receptor, Sprouty2, and Spred.

10. The composition of Claim 6, wherein the disease is osteoarthritis.
